# FASCICULE DE BREVET EUROPEEN

(11) **EP 1 515 696 B1**
(45) Date de publication et mention de la délivrance du brevet: **13.08.2008**
(21) Numéro de dépôt: 03761638.0
(22) Date de dépôt: 25.06.2003
(51) Int. Cl.: A61K 8/49, A61K 8/97, A61Q 19/00

(54) **UTILISATION D'ISOFLAVONES POUR FAVORISER L'AMINCISSEMENT**
VERWENDUNG VON ISOFLAVONEN ZUR FÖRDERUNG DES SCHLANKHEITSEFFEKTS
USE OF ISOFLAVONES FOR PROMOTING SLIMMING

(30) Priorité: 27.06.2002 FR 0207995
(43) Date de publication de la demande: 23.03.2005
(73) Titulaire: Laboratoires Expanscience, 92400 Courbevoie (FR)
(72) Inventeur: MSIKA, Philippe, F-78000 Versailles (FR); PICCARDI, Nathalie, F-38120 Saint Egrève (FR)
(74) Mandataire: Warcoin, Jacques
(86) Numéro de dépôt international: PCT/FR2003/001956
(87) Numéro de publication internationale: WO 2004/002435

(56) Documents cités:
- EP-A- 0 829 261
- WO-A-01/64177
- WO-A-01/74345
- WO-A-99/47118
- WO-A-02/087700
- DE-A- 10 009 423
- FR-A- 2 822 068
- US-A1- 2002 160 064
- PATENT ABSTRACTS OF JAPAN vol. 1998, no. 09 (C, & JP 10 087486 A (SEKIYA KEIZO), 7 avril 1998 (1998-04-07)
- SCHMIDT ET AL.: "Genistein, a new cosmetic ingredient derived from soy" SÖFW-JOURNAL, vol. 127, no. 10, 12 octobre 2001 (2001-10-12), pages 22-27, XP002235454 DE

## Description

La présent invention est relative à l'utilisation d'isoflavones et d'un extrait de fleurs de Sophora Japonica pour la préparation de compositions topiques utiles pour favoriser l'amincissement et à la méthode de traitement cosmétique associée.

L'amincissement dans le cadre de la présente invention passe préférentiellement par la lutte contre la surcharge pondérale localisée.
Cette surcharge pondérale localisée se matérialise sous forme de graisses, dont la quantité et la répartition diffèrent en fonction du sexe. Ainsi, le tissu adipeux représente 20 à 30% du poids corporel chez la femme et 10 à 15% chez l'homme. La graisse sous-cutanée est deux fois plus épaisse chez la femme que chez l'homme. Chez l'homme les graisses s'accumulent autour et au-dessus de la ceinture (répartition androïde facteur de risque métabolique) et au-dessous de la ceinture, dans la région glutéo-fémoralé chez la femme (répartition gynoïde, non corrélée à un risque vasculaire). L'une des caractéristiques de cette graisse accumulée en bas du corps est d'être difficilement mobilisable. Elle est destinée à assurer les besoins énergétiques de la reproduction (grossesse et, surtout, allaitement) et constitue ainsi le plus important réservoir énergétique de l'organisme.
Au niveau cellulaire, les adipocytes sont des cellules sphériques sont l'espace intracellulaire est comblé par une large vacuole remplie de triglycérides. Les adipocytes peuvent changer rapidement de volume. En effet, ces cellules peuvent atteindre, selon les circonstances 40 µm à 120 µm de diamètre, ce qui correspond à une augmentation de 27 fois en volume. Dans certains cas extrêmes, cette augmentation peut aller jusqu'à 40 fois. Ainsi, l'adipocyte est le principal acteur énergétique de l'organisme puisqu' il est capable de stocker (captation ou lipogenèse) ou, inversement, de mobiliser (lipolyse) rapidement les triglycérides, sources énergétiques majeures de l'organisme.
La lipogenèse passe par la synthèse des triacylglycérols qui résulte de l'estérification du glycérol-3-phosphate par les acides gras activés; à l'inverse, la lipolyse correspond à l'hydrolyse des triacylglycérols stockés, en glycérol et en acides gras. Différents mécanismes ont été mis en lumière, qui contrôlent la lipolyse et la lipogenèse qui font par exemple intervenir des récepteurs tels que les récepteurs alpha-2 et/ou béta-1 et -2, les récepteurs de l'adénosine de type A1, de la prostaglandine E2, Y2 de type YY et du neuropeptide NPY, mais aussi les hormones sexuelles.

Ainsi, la connaissance des mécanismes de contrôle de la lipolyse et de la lipogenèse adipocytaires s'est très nettement améliorée. Toutefois, des actifs amincissants sont toujours recherchés car les actifs amincissants connus ne sont pas totalement satisfaisants. Il existe donc à ce jour une réelle demande pour l'élaboration de compositions topiques permettant de favoriser efficacement l'amincissement.

On connaît deux grands types d'actifs amincissants : les lipolytiques (agissent au niveau de l'élimination des surcharges lipidiques) et les liporéducteurs (luttent contre la formation des graisses).

### a) Les lipolytiques

➢ La caféine (que l'on retrouve dans de nombreux végétaux : thé vert, graines de guarana): inhibe la phosphodietérase, assurant ainsi un taux d'AMPc intracellulaire optimal, stimule les récepteurs β et inhibe la lipoprotéine lipase ;
➢ Le rhodystérol (extrait d'une algue rouge) : active les récepteurs a et favorise la pénétration de la caféine;
➢ La palmitoyl-carnitine : accélère la combustion des acides gras, en améliorant leur captation par les mitochondries;
➢ Les bioactifs alpha et gamma (issus respectivement d'une bactérie marine et d'un champignon) : bloquent les récepteurs α 2 et NPY;
➢ L'escine et le ginkgo biloba : α 2 bloqueurs;
➢ La sphingosine : limite la pénétration du glucose

### b) Les liporéducteurs

➢ *Andiroba* (triterpènes) et *Carcina Cambogia* : bloquent la transformation des pré-adipocytes en adipocytes
➢ La rutine : (extraite de *Ruta graveolens*) : isole le glucose et empêche son association avec les acides gras libres.

A ces actifs spécifiques, peuvent s'ajouter des actifs désinfiltrants et des veinotoniques, qui sont souvent associés aux actifs amincissants.

### Désinfiltrants :

❖ Viburnum (drainant, décongestionnant, active effet caféine, anti-radicalaire, raffermissant)
❖ Lierre (anti-inflammatoire, anti-oedémateux, analgésique)
❖ L'arnica (anti-oedémateux, apaisant)
❖ La pisolle (anti-radicalaire, accélère le drainage)
❖ Pensée sauvage (rôle important dans l'équilibre hydrique)
❖ Fucus vesiculosus (anti-oedémateux, apaisant)

### Veinotoniques :

❖ Ruscus (action vitaminique P, tonique vasculaire)
❖ Ginkgo biloba (lutte contre la stase vasculaire et capillaire)
❖ L'escine (amélioration du tonus veineux, modifie la perméabilité capillaire)

Enfin, les formulations comprenant ces actifs amincissants connus peuvent être complétées par des actifs restructurant et lissant qui luttent contre le relâchement de la peau.

On a maintenant trouvé que l'application d'une composition topique comprenant une ou plusieurs isoflavones et un extrait de fleurs de Sophora Japonica a une action amincissante et permet notamment de lutter contre la surcharge pondérale localisée.

La demande de brevet W001/64177 (HENKEL KGAA) divulgue l'utilisation d'une composition comprenant une flavone, une isoflavone, ou la glycosyle et éventuellement une substance stimulant et/ou dépolarisant les fibres nerveuses pour les traitements cosmétiques de la cellulite ou de l'affermissement de la peau.

Dans l'article Cosmetics, D SCHMID et al., SÖFW-Journal, 127. Jahrgang 10-200, « Genisteine, a new cosmetic ingredient derived from soy", l'action anti-cellulite de certaines isoflavones est décrite. Cependant, la cellulite est une affection bien différente de la surcharge pondérale localisée.

Ainsi, la cellulite est définie comme un désordre localisé métabolique des tissus sous-cutanés qui provoque une altération des formes du corps. A l'inverse, la surcharge pondérale est due à une hypertrophie ou hyperplasie des adipocytes (augmentation de la lipogenèse), diminution de la lipolyse. La différenciation cellulite-amincissment apparaît également au vu du fait que la cellulite touche le derme (tissu sous-cutané) des femmes uniquement alors que la surcharge pondérale (les adipocytes) touche l'ensemble des deux sexes. De plus, la cellulite apparaît chez les femmes même sans surcharge pondérale ; c'est un problème d'architecture derme/hypoderme.

La demande de brevet EP 829261 a trait à des compositions comprenant des isoflavones pour leur effet réducteur de poids par la dégradation de graisses accumulées dans les cellules graisseuses. Toutefois, le mode d'administration topique n'est pas évoqué dans cette demande de brevet.

La présente invention a ainsi pour objet l'utilisation d'isoflavones et d'un extrait de fleurs de Sophora Japonica pour la préparation de compositions topiques utiles pour favoriser l'amincissement et à la méthode de traitement cosmétique associée.

Les «isoflavones» utilisables selon la présente invention peuvent être des substances naturelles extraites de produits naturels, notamment à partir de végétaux tels que le soja, le trèfle, le lupin, les pépins de pomme etc. Bien souvent les compositions topiques selon la présente invention contiennent, à titre d'isoflavones un mélange de différentes isoflavones, mais elles peuvent également être présentes sous forme pure dans le cadre de la présente invention. Par ailleurs, on distingue les formes aglycones des isoflavones et les formes glycosylées de ces dernières. Ces diverses formes se trouvent le plus souvent en mélange. Elles sont illustrées par les formules suivantes.

Formes aglycones, de formule : 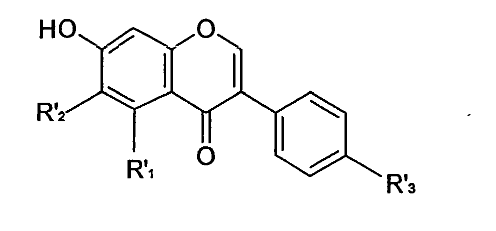 dans laquelle R'₁ représente un atome d'hydrogène ou un groupe hydroxy, R'₂ représente un atome d'hydrogène ou un groupe méthoxy et R'₃ représente un groupe hydroxy.

Avantageusement, selon la présente invention, R'₁, R'₂ et R'₃ représentent :

| **R'₁** | **R'₂** | **R'₃** | **Nom du composé** |
|---|---|---|---|
| H | H | OH | Daidzéine |
| OH | H | OH | Génistéine |
| H | OCH₃ | OH | Glycitéine |

Formes glycosylées, de formule : 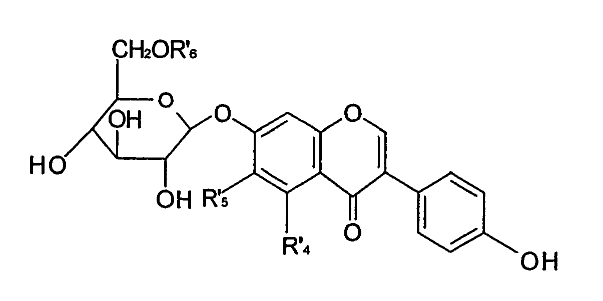 dans laquelle R'₄ représente un atome d'hydrogène ou un groupe hydroxy, R'₅ représente un atome d'hydrogène ou un groupe méthoxy et R'₆ représente un atome d'hydrogène.

Avantageusement, selon la présente invention R'₄, R'₅ et R'₆ représentent :

| **R'₄** | **R'₅** | **R'₆** | **Nom du composé** |
|---|---|---|---|
| H | H | H | Daidzine |
| OH | H | H | Génistine |
| H | OCH₃ | H | Glycitine |

Les formes glycosylées des isoflavones sont les plus abondantes dans la nature.

On préfère, à titre d'isoflavones, les isoflavones naturelles telles que la génistéine (1), la daidzéine ou la glycitéine.

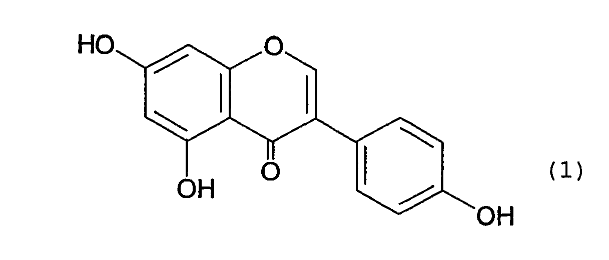

En particulier, la génistéine ou 4,5,7-trihydroxyisoflavone utilisable selon la présente invention peut être un produit d'origine végétale et notamment de soja, titrant 85 à 90 % en poids de génistéine, notamment le produit commercialisé par la société Buckton Scott sous le nom "génistéine titrée à 85%".

Les isoflavones peuvent être utilisées seules ou en mélange dans le cadre de la présente invention.

L'application topique d'une composition d'une ou plusieurs isoflavones peut s'avérer particulièrement avantageuse chez la femme enceinte ou la femme ayant accouché depuis moins de 6 mois. La présente invention a ainsi également pour objet une méthode de traitement cosmétique pour favoriser l'amincissement et notamment pour lutter contre la surcharge pondérale localisée chez la femme enceinte ou la femme ayant accouché depuis moins de 6 mois. En effet, l'un des avantages de compositions utilisables dans le cadre de la présente invention est de ne pas nécessiter la présence d'alcool pour la formulation, qui est contre indiqué pour les femmes enceintes et en allaitement du fait de sa toxicité. En effet, la caféine, actif amincissant très couramment utilisé nécessite une solubilisation dans l'alcool, qui est ainsi évitée dans le cadre de la présente invention.

Par l'expression « amincissement » ou « lutte contre la surcharge pondérale localisée», on entend selon la présente invention une action permettant d'éviter ou à tout le moins de réduire la formation de graisses sous-cutanées telles que décrites précédemment. Cette action se traduit notamment par une diminution des surcharges ou réserves disgracieuses, par un affinement de la silhouette, par une accélération de l'élimination des excédents, par une meilleure définition du contour de corps ou encore une silhouette resculptée.

Par méthode de « traitement cosmétique pour lutter contre la surcharge pondérale localisée » on entend, selon la présente invention, la mise en oeuvre d'un traitement cosmétique permettant de mesurer de manière visible l'action décrite ci-dessus.

Ainsi, une composition topique comprenant une ou des isoflavones et un extrait de fleurs de Sophara Japonica utilisée selon l'invention peut être appliquée sur les zones de la peau susceptibles de former ces surcharges pondérales localisées, à savoir des zones où ces surcharges sont déjà formées ou en cours de formation.
A titre d'exemple, une composition contenant de la génistéine que l'on peut mettre en oeuvre dans le cadre de la présente invention peut contenir entre 0,0085 et 8,5 % de génistéïne en poids par rapport au poids total de la composition, soit pour une solution titrant 85 à 90 % en poids de génistéïne, entre 0,01 et 10% en poids de cette solution par rapport au poids total de la composition.

Plus généralement, une composition contenant une ou plusieurs isoflavones que l'on peut mettre en oeuvre dans le cadre de la présente invention peut contenir entre 0,01 et 10% en poids d'isoflavone(s) par rapport au poids total de la composition, et de préférence de 0,1 à 3%.
La composition qui permet la mise en oeuvre de l'invention comprend un support cosmétiquement acceptable, c'est à dire un support compatible avec la peau et peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et ou non-ionique.
Cette composition peut être plus ou moins fluide et avoir l'aspect d'une crème blanche ou colorée, d'une pommade, d'un lait, d'une lotion, d'un sérum, d'une pâte, d'une mousse ou d'un gel.
Elle peut éventuellement être appliquée sur la peau sous forme d'aérosol. Elle peut également se présenter sous forme solide, et par exemple sous forme de stick.
La composition de l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les agents chélateurs, les absorbeurs d'odeur et les matières colorantes. Les quantités de ces différents adjuvants sont celles classiquement utilisées dans les domaines considérés, et par exemple de 0,01 à 20% du poids total de la composition. Ces adjuvants, selon leur nature, peuvent être introduits dans la phase grasse, dans la phase aqueuse, dans les vésicules lipidiques et
ou dans les nanoparticules.
Lorsque la composition de l'invention est une émulsion, la proportion de la phase grasse peut aller de 5 à 80% en poids, et de préférence de 5 à 50% du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30% en poids, et de préférence de 0,5 à 20% du poids total de la composition.
Comme huiles utilisables dans les compositions permettant de mettre en oeuvre l'invention, on peut citer les huiles minérales, les huiles d'origine végétale (huile d'abricot, huile de tournesol, de prune), les huiles d'origine animale, les huiles de synthèse, les huiles siliconées et les huiles fluorées (perfluoropolyéthers). On peut aussi utiliser comme matières grasses des alcools gras (alcool cétylique), des acides gras, des cires (cire d'abeilles).
Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stearate de glycéryle et le tristéarate de sorbitane.
Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles' on peut citer les argiles modifiées comme les bentones, les sels métalliques d'acides gras, la silice hydrophobe et les polyethylenes.

La composition utilisée selon l'invention peut contenir d'autres actifs à action amincissante comme les lipolytiques et les liporéducteurs tels que décrits en introduction.

L'invention concerne ainsi l'utilisation d'isoflavones et d'un extrait de fleurs et Sophora Japonica pour la préparation de compositions topiques utiles pour favoriser l'amincissement et notamment pour lutter contre la surcharge pondérale localisée, caractérisée en ce que l'on applique, de façon simultanée, séparée ou étalée dans le temps une ou plusieurs isoflavones ainsi qu'un ou deux actifs amincissants de type lipolytique et/ou un ou deux actifs amincissants de type liporéducteur.

L'actif amincissant de type lipolytique peut être choisi parmi : la caféine, le rhodystérol, la palmitoyl-carnitine, les bioactifs alpha et gamma, l'escine, le ginkgo biloba et la sphingosine. L'actif amincissant de type liporéducteur peut être choisi parmi : l'andiroba, la Garcinia Cambogia, la rutine.

On peut également appliquer de façon simultanée, séparée ou étalée dans le temps un ou deux actifs désinfiltrants ou veinotoniques en plus de l'application de la composition utilisée selon l'invention. Les actifs désinfiltrants ou veinotoniques peuvent être choisis parmi : le viburnum, le lierre, l'arnica, la pisolle, la pensée sauvage, le *Fucus vesiculosus,* la *ruscus*, le ginkgo biloba et l'escine.
La composition utilisée selon l'invention comprend:
- Un extrait de fleurs *sophora japonica* : cet extrait est riche en flavonoïdes (anti radicalaire) et en rutine. Cet actif favorise la microcirculation, facilitant et activant le drainage et la désinfiltration des tissus ;
- et peut en autre comprendre d'autres actifs tels que: - L'extrait de *centella asiatica* : Extrait de *centella,* plante originaire de l'Afrique de l'est et du Madagascar. Cet actif contient des terpènes (asiaticosides, de l'acide asiatica et de l'acide madécassique), aux propriétés drainantes, désinfiltrantes et raffermissantes sur les tissus. Il est notamment utilisé dans les produits amincissants mais aussi dans les produits anti-vergetures, antirides et cicatrisants ; 0 à 5% d'extrait de *centella* peut ainsi être présent dans une composition amincissante ;
- « Hydrolyzed Soy Protein » : protéine de soja qui est un élastorégulateur. Ces peptides du soja peuvent être tout peptide obtenu par hydrolyse de protéines extraites du soja, selon des conditions opératoires connues de l'homme du métier, en d'autres termes tout hydrolysat de protéine du soja. Les peptides de soja, qui sont décrits dans la demande de brevet WO 00/19974 sont particulièrement adaptés pour être introduits dans les compositions utilisées dans le cadre de la présente invention. Cet actif permet la restauration des mécanismes de renouvellement cellulaire, active la synthèse des éléments structuraux de la matrice extracellulaire et possède une action restructurante, régénérante et raffermissante ; 0 à 5% de protéine de soja peut ainsi être présent dans une composition amincissante;
- des actifs anti-âge, parmi lesquels on peut citer les furanes d'avocat, le rétinol et ses dérivés, la vitamine C ou les insaponifiables de soja etc...

L'association des deux actifs isoflavone prise seule ou en mélange et extrait de fleurs de *Sophora Japonica* est particulièrement préférée

Une composition contenant cette association peut contenir de 0,01 à 20 % d'extrait de fleurs de *Sophora Japonica.*

Les exemples suivant illustrent la présente invention.

**Exemple 1 : Gel restructurant**

| Ingrédients (INCI - EU) | % p/p |
|---|---|
| Aqua | QSP |
| PEG-6 | 3,6000 |
| Butylene glycol | 2,7000 |
| Dextrin | 1,8600 |
| Phenyl Trimethicone | 1,2000 |
| Acrylates/C10-30 alkyl acrylate crosspolymer | 0,6000 |
| Système conservateur | QSP |
| Dimethicone/Phenyl vinyl Dimethicone Crosspolymer | 0,3000 |
| Parfum | QS |
| *Sophora Japonica* Flower Extract | 0,01 à 20 |
| Xanthan Gum | 0,1500 |
| PPG 26-Buteth-26 | 0,1100 |
| Genosten® 4000 ⁽¹⁾ | 1 à 10 |
| Hydrolyzed Soy Protein | 0,1000 |
| Glucose | 0,0800 |
| PEG 40 Hydrogenated Castor Oil | 0,0700 |
| Sorbitol | 0,0400 |
| *Centella Asiatica* Extract | 0,01 à 5 |
| Citric Acid | 0,0200 |
| *Enteromorpha Compressa* Extract | 0,01 à 5 |
| Colorant | QS |
| Sodium Hydroxide | QSP pH = 4,5 à 6,5 |

| | |
|---|---|
| QS = quantité suffisante QSP = quantité suffisante pour ⁽¹⁾ commercialisé par la société Nutrinov Extrait de soja riche en isoflavones obtenu par extraction physique : 4% isoflavones dont : Daidzine : 0,28 g o. Malonyl daidzine : 0,93 g Génistine : 0,28 g O Malonyl génistine : 2,50 g Génistéine : 0,02 g Dadzéine : 0 g | |

### Exemple 2 : Gel restructurant

| Ingrédients (INCI - EU) | % p/p |
|---|---|
| Aqua | QSP |
| PEG-6 | 3,6000 |
| Butylene glycol | 2,7000 |
| Dextrin | 1,8600 |
| Phenyl Trimethicone | 1,2000 |
| Acrylates/C10-30 alkyl acrylate crosspolymer | 0,6000 |
| Système conservateur | QSP |
| Dimethicone/Phenyl vinyl Dimethicone Crosspolymer | 0,3000 |
| Parfum | QS |
| *Sophora Japonica* Flower Extract | 0,01 à 20 |
| Xanthan Gum | 0,1500 |
| PPG 26-Buteth-26 | 0,1100 |
| 4,5,7-Trihydroxyisoflavone | 0,01 à 10 |
| Hydrolyzed Soy Protein | 0,1000 |
| Glucose | 0,0800 |
| PEG 40 Hydrogenated Castor Oil | 0,0700 |
| Sorbitol | 0,0400 |
| *Centella Asiatica* Extract | 0,01 à 5 |
| Citric Acid | 0,0200 |
| *Enteromoxpha Compressa* Extract | 0,01 à 5 |
| Colorant | QS |
| Sodium Hydroxide | QSP pH = 4,5 à 6,5 |

| | |
|---|---|
| QS = quantité suffisante QSP = quantité suffisante pour | |

### Exemple 3 : crème huile dans eau

| Ingrédients (INCI - EU) | % p/p |
|---|---|
| Aqua | QSP |
| Squalane | 5,00 |
| Petrolatum | 5,00 |
| Glycerin | 5,00 |
| Isodecyl Neopentanoate | 5,00 |
| Pentaerythrityl Tetraethylhexanoate | 5,00 |
| Cyclomethicone | 4,00 |
| Cetearyl Alcohol | 3,00 |
| Myristyl Myristate | 2,00 |
| Laureth-23 | 2,00 |
| Silica | 2,00 |
| Heptadecadienyl Furan | 0,1 à 10 |
| Beeswax | 1,00 |
| Sclerotium Gum | 1,00 |
| PEG-6 | 1,00 |
| Polyacrylamide | 0,80 |
| Glyceryl Stearate | 0,70 |
| Dimethiconol | 0,70 |
| Cetearyl Glucoside | 0,60 |
| C13-14 Isoparaffin | 0,40 |
| Citric Acid | 0,14 |
| Laureth-7 | 0,10 |
| Mélange quimdis ⁽²⁾ | 0,01 à 10 |
| Cafeine | 0,1 à 10 |
| *Enteromorpha Compressa* Extract | 0,01 à 5 |
| *Garcinia Cambogia* Extract | 0,01 à 10 |
| Ginkgo Biloba Extract | 0,01 à 10 |
| *Sophora Japonica* Flower Extract | 0,01 à 20 |
| Hydrolyzed Soy Protein | 0,01 à 10 |
| Preservative system | QS |
| Fragrance | QS |

| | |
|---|---|
| QS = quantité suffisante QSP = quantité suffisante pour ⁽²⁾ commercialisé par la société Quimdis Mélange d'isoflavones entre 7 et 17 % 4 à 8 % Glycosides de dadzéine 2 à 5 % Glycosides de glycitine 1 à 4 % Glycosides de génistéine | |

### Exemple 4 : crème huile dans eau

| Ingrédients (INCI - EU) | % p/p |
|---|---|
| Aqua | QSP |
| Squalane | 5,00 |
| Petrolatum | 5,00 |
| Glycerin | 5,00 |
| Isodecyl Neopentanoate | 5,00 |
| Pentaerythrityl Tetraethylhexanoate | 5,00 |
| Cyclomethicone | 4,00 |
| Cetearyl Alcohol | 3,00 |
| Myristyl Myristate | 2,00 |
| Laureth-23 | 2,00 |
| Silica | 2,00 |
| Heptadecadienyl Furan | 0,1 à 10 |
| Beeswax | 1,00 |
| Sclerotium Gum | 1,00 |
| PEG-6 | 1,00 |
| Polyacrylamide | 0,80 |
| Glyceryl Stearate | 0,70 |
| Dimethiconol | 0,70 |
| Cetearyl Glucoside | 0,60 |
| C13-14 Isoparaffin | 0,40 |
| Citric Acid | 0,14 |
| Laureth-7 | 0,10 |
| 4,5,7-Trihydroxyisoflavone | 0,01 à 10 |
| Cafeine | 0,1 à 10 |
| *Enteromorpha Compressa* Extract | 0,01 à 5 |
| *Garcinia Cambogia* Extract | 0,01 à 10 |
| Ginkgo Biloba Extract | 0,01 à 10 |
| *Sophora Japonica* Flower Extract | 0,01 à 20 |
| Hydrolyzed Soy Protein | 0,01 à 10 |
| Preservative system | QS |
| Fragrance | QS |

| | |
|---|---|
| QS = quantité suttisante QSP = quantité suffisante pour | |

### Exemple 5 : crème eau dans huile

| Ingrédients (INCI - EU) | % p/p |
|---|---|
| Aqua | QSP |
| Hydrogenated Polyisobutene | 7,00 |
| Isocetyl Stearate | 7,00 |
| Cyclomethicone | 4,80 |
| Glycerin | 4,00 |
| Mineral Oil | 3,00 |
| Zinc Oxide | 3,00 |
| Butylene Glycol | 2,00 |
| Isononyl Isononanoate | 2,00 |
| Beeswax | 2,00 |
| Cetyl Dimethicone Copolyol | 1,70 |
| Polyglyceryl-4 Isostearate | 1,65 |
| Hexyl laurate | 1,65 |
| Disodium tartrate | 1,60 |
| Sodium Chloride | 1,00 |
| PEG-6 | 1,00 |
| 4,5,7-Trihydroxyisoflavone | 0,01 à 10 |
| Retinyl palmitate | 0,01 à 10 |
| *Enteromorpha Compressa* Extract | 0,01 à 5 |
| *Sophora Japonica* Flower Extract | 0,01 à 20 |
| *Centella Asiatica* Extract | 0,01 à 5 |
| Hydrolyzed soy protein | 0,01 à 10 |
| Preservative system | QS |
| Fragrance | QS |

| | |
|---|---|
| QS = quantité suffisante QSP = quantité suffisante pour | |

### Exemple 6 : stick

| Ingrédients (INCI - EU) | % p/p |
|---|---|
| Castor Oil | QSP |
| Oleyl Alcohol | 20,00 |
| Hydrogenated Palm Kernel Oil | 17,00 |
| Candelilla Wax | 11,00 |
| Polyglyceryl-3 Beeswax | 10,00 |
| Mineral Oil | 9,57 |
| Heptadecadienyl Furan | 0,1 à 1 |
| 4,5,7-Trihydroxyisoflavone Quaternium-18 Hectorite | 0,01 à 1,10 |
| Titanium Dioxide | 1,00 |
| Tocopheryl Acetate | 0,50 |
| Propylene Carbonate | 0,33 |
| Fragrance | QS |
| Retinol | 0,01 |
| *Enteromorpha Compressa* Extract | 0,01 à 5 |
| *Sophora Japonica* Flower Extract | 0,01 à 20 |
| *Centella Asiatica* Extract | 0,01 à 5 |
| Hydrolyzed soy protein | 0,01 à 10 |

| | |
|---|---|
| QS = quantité suffisante QSP = quantité suffisante pour | |

### Exemple 7 : gel crème

| Ingrédients (INCI - EU) | % p/p |
|---|---|
| Aqua | QSP |
| Cyclomethicone | 5,40 |
| Octyl Palmitate | 5,00 |
| Hydrogenated Coco-glycerides | 3,00 |
| Arachidyl Behenyl Alcohol | 2,55 |
| Propylene Glycol | 2,50 |
| Isodecyl Neopentanoate | 2,00 |
| Glyceryl Stearate | 1,70 |
| Cetyl Alcohol | 1,30 |
| Stearic Acid | 1,00 |
| PEG-6 | 1,00 |
| Beeswax | 0,40 |
| C13-14 Isoparaffin | 0,40 |
| Butylene Glycol | 0,16 |
| Glycerin | 0,16 |
| Cetearyl Alcohol | 0,10 |
| Cetyl Palmitate | 0,10 |
| Cocoglycerides | 0,10 |
| Laureth-7 | 0,10 |
| Mélange novasoy ⁽³⁾ | 0,01 à 10 |
| *Enteromorpha Compressa* Extract | 0,01 à 5 |
| *Sophora Japonica Flower* Extract | 0,01 à 20 |
| *Centella Asiatica* Extract | 0,01 à 5 |
| Hydrolyzed soy protein | 0,01 à 10 |
| Preservative system | QS |
| Fragrance | QS |

| | |
|---|---|
| QS = quantité suffisante QSP = quantité suffisante pour ⁽³⁾ Commercialisé par la société ADM Mélange d'isoflavones à minimum 30 % Ratio génistéine/daidzine/glycitine : 1.3/1.0/0.3 Exemple de dosage : Génistéine : 20,80 % Glycitine : 3,80 % Daideine : 12,00 % | |

### Exemple 8 : gel crème

| Ingrédients (INCI - EU) | % p/p |
|---|---|
| Aqua | QSP |
| Cyclomethicone | 5,40 |
| Octyl Palmitate | 5,00 |
| Hydrogenated Coco-glycerides | 3,00 |
| Arachidyl Behenyl Alcohol | 2,55 |
| Propylene Glycol | 2,50 |
| Isodecyl Neopentanoate | 2,00 |
| Glyceryl Stearate | 1,70 |
| Cetyl Alcohol | 1,30 |
| Stearic Acid | 1,00 |
| PEG-6 | 1,00 |
| Beeswax | 0,40 |
| C13-14 Isoparaffin | 0,40 |
| Butylene Glycol | 0,16 |
| Glycerin | 0,16 |
| Cetearyl Alcohol | 0,10 |
| Cetyl Palmitate | 0,10 |
| Cocoglycerides | 0,10 |
| Laureth-7 | 0,10 |
| 4,5,7-Trihydroxyisoflavone | 0,01 à 10 |
| *Enteromorpha Compressa* Extract | 0,01 à 5 |
| *Sophora Japonica Flower* Extract | 0,01 à 20 |
| *Centella Asiatica* Extract | 0,01 à 5 |
| Hydrolyzed soy protein | 0,01 à 10 |
| Preservative system | QS |
| Fragrance | QS |

| | |
|---|---|
| QS = quantité suffisante QSP = quantité suffisante pour | |

### Exemple 9 : spray

| Ingrédients (INCI - EU) | % p/p |
|---|---|
| Aqua | QSP |
| Glycerin | 4,00 |
| Montmorillonite | 3,00 |
| PEG | 3,00 |
| Glycine | 0,30 |
| Citric acid | 0,09 |
| 4,5,7-Trihydroxyisoflavone | 0,01 à 10 |
| *Enteromorpha Compressa* Extract | 0,01 à 5 |
| *Sophora Japonica* Flower Extract | 0,01 à 20 |
| *Centella Asiatica* Extract | 0,01 à 5 |
| Hydrolyzed soy protein | 0,01 à 10 |
| Preservative system | QS |
| Fragrance | QS |

| | |
|---|---|
| QS = quantité suffisante QSP = quantité suffisante pour | |

### Exemple 10 : Résumé du test d'utilisation du gel restructurant selon l'exemple 1

On a cherché dans ce test à apprécier l'efficacité et l'acceptabilité du gel restructurant selon l'exemple 1, après applications répétées, 2 fois par jour sur une période de 8 semaines, dans les conditions normales d'utilisation, par un groupe de 92 à 100 consommatrices ayant accouché depuis moins de 3 mois et/ou à la peau du corps (ventre et cuisses) présentant une surcharge lipidique.

### PANELISTES

L'analyse des résultats a porté sur un panel de 92 (T 2 mois) à 100 (T 1 mois) consommatrices, dont les caractéristiques physiques sont présentées ci-dessous.

**Panel à T 1 mois (100 consommatrices)**

| Age | Nature de la peau du corps | Peau « sensible» |
|---|---|---|
| 19 à 49 ans (moy. :30 ans) | - sèche : 44 % | 35 % |
| | - normale : 36 % | |
| | - mixte sèche-normale : 20 % | |

A noter, que 48 panélistes venaient d'avoir leur première grossesse, que 44 avaient eu des grossesses antécédentes au nombre de 2 (23), de 3 (20) et de 4 (1)

**Panel à T 2 mois (92 consommatrices)**

| Age | Nature de la peau du corps | Peau «sensible» |
|---|---|---|
| 19 à 49 ans | - sèche : 45 % | |
| (moy. :30 ans) - | normale : 33,7 % | 37 % |
| | - mixte sèche-normale : 20 % | |

A noter, que 48 panélistes venaient d'avoir leur première grossesse, que 40 avaient eu des grossesses antécédentes au nombre de 2 (21), de 3 (19).

### PROTOCOLE

Le gel restructurant étudié a été appliqué 2 fois par jour en moyenne (matin et soir), pendant 1 à 2 mois, sur le corps (cuisses, hanches, ventre et fesses en particulier), par les consommatrices, à leur domicile, dans les conditions normales d'utilisation, à la place de celui qu'elles utilisent généralement.

Les questionnaires, adaptés à la nature du produit, ont été complétés à la fin du test (T1 et T2 mois) puis renvoyés par courrier, et comportaient notamment les questions listées ci-dessous relatives à l'action de lutte contre la surcharge pondérale :

Le gel restructurant selon l'exemple 1, appliqué pendant 2 mois par les 92 à 100 femmes décrites ci-dessus a été bien apprécié puisque 87 % des panélistes l'ont jugé globalement « agréable » à « très agréable » et 67 % globalement « bon » à « très bon ». Les caractéristiques de performance suivantes ont été mises en évidence.

| Performance | Effets à T 1 mois (100 consommatrices) | Effets à T 2 mois (92 consommatrices) |
|---|---|---|
| | Exprimé en % du panel | |
| Surcharges disgracieuses diminuées | 76 | 86 |
| Réserves disgracieuses diminuées | 70 | 80 |
| Silhouette affinée | 75 | 87 |
| «accélère» l'élimination des excédents | 74 | 86 |
| Modèle le corps | 70 | 90 |
| Contours du corps mieux définis | 77 | 68 |
| La silhouette est resculptée | 60 | - |

## Revendications

1. Utilisation d'isoflavones sous forme aglycones ou glycosylées et un extrait de fleurs de Sophora Japanica pour la préparation de compositions topiques utiles pour favoriser l'amincissement.

2. Utilisation selon la revendication 1, **caractérisée en ce que** les compositions topiques sont utiles pour lutter contre la surcharge pondérale localisée.

3. Utilisation selon la revendication 1, **caractérisée en ce que** les compositions topiques sont utiles pour affiner la silhouette, accélérer l'élimination des excédents, mieux définir le contour du corps et/ou resculpter la silhouette.

4. Utilisation selon l'une quelconque des revendications 1 à 3, **caractérisée en ce que** l'on applique, de façon simultanée, séparée ou étalée dans le temps, une ou plusieurs isoflavones ainsi que un
ou deux actifs amincissants de type lipolytique et/ou à un ou deux actifs amincissants de type liporéducteur.

5. Utilisation selon la revendication 4, **caractérisée en ce que** l'actif amincissant de type lipolytique est choisi parmi : la caféine, le rhodystérol, la palmitoyl-carnitine, les bioactifs alpha et gamma, l'escine, le ginkgo biloba et la sphingosine.

6. Utilisation selon la revendication 4 ou 5,
**caractérisée en ce que** l'actif amincissant de type liporéducteur est choisi parmi : *l'andiroba,* la *Garcinia Cambogia* et la rutine.

7. Utilisation selon l'une quelconque des revendications là 6, **caractérisée en ce que** l'on applique en outre de façon simultanée, séparée ou étalée dans le temps, un ou deux actifs désinfiltrants ou veinotoniques choisis parmi : le Viburnum, le lierre, l'arnica, la pisolle, la Pensée sauvage, le *Fucus vesiculosus,* le *Ruscus,* le Ginkgo biloba et l'escine.

8. Utilisation selon l'une quelconque des revendications 1 à 7, **caractérisée en ce que** l'on applique en outre de façon simultanée, séparée ou étalée dans le temps, un extrait de *Centella Asiatica* et/ou des protéines de soja.

9. Utilisation selon l'une quelconque des revendications 1 à 8, **caractérisée en ce que**, à titre d'isoflavones, on choisi parmi : la génistéine, la daidzéine, la glycitéine ou un mélange de ces derniers ; ces dernières étant sous forme aglycones
ou glycosylées.

10. Utilisation selon la revendication 8, **caractérisée en ce que** la génistéine est d'origine végétale de soja.

11. Méthode de traitement cosmétique pour favoriser l'amincissement, **caractérisée en ce que** l'on applique par voie topique une composition cosmétique comprenant une ou plusieurs isoflavones sous forme aglycones ou glycosylées et un extrait de fleurs de Sophora Japonica

12. Méthode de traitement cosmétique pour lutter contre la surcharge pondérale localisée, **caractérisée en ce que** l'on applique par voie topique une composition comprenant une ou plusieurs isoflavones sous forme aglycones ou glycosylées, et un extrait de fleurs de Sophora Japonica

13. Méthode de traitement cosmétique pour affiner la silhouette, accélérer l'élimination des excédents, mieux définir le contour du corps et/ou resculpter la silhouette, **caractérisée en ce que** l'on applique par voie topique une composition comprenant une ou plusieurs isoflavones, sous forme aglycones ou glycosylées, et un extrait de fleurs le Sophora Japonica

14. Méthode de traitement cosmétique selon l'une quelconque des revendications 11 à 13, **caractérisée en ce que** l'on applique par voie topique sur les zones de la peau susceptibles de former des surcharges pondérales localisées, de façon simultanée, préparée ou étalée dans le temps, une ou plusieurs isoflavones ainsi que le ou les actifs tels que définis dans l'une quelconque des revendications 4 à 8.

15. Méthode de traitement cosmétique selon l'une quelconque des revendications 11 à 14, **caractérisée en ce que**, à titre d'isoflavones, on choisi parmi : la génistéine, la daidzéine, la glycitéine ou un mélange de ces derniers, ces dernières étant sous forme aglycones ou glycosylées.

16. Méthode de traitement cosmétique selon la revendication 15, **caractérisée en ce que** la génistéine est d'origine végétale de soja.

17. Méthode de traitement cosmétique selon l'une quelconque des revendications 11 à 16, **caractérisée en ce que** l'on traite les femmes enceintes ou les femmes ayant accouché depuis moins de 6 mois.

## Claims

1. Use of isoflavones in aglycone or glycosylated form of and an extract of *Sophora japonica* flowers for the preparation of topical compositions that are useful for promoting slimming.

2. Use according to Claim 1, **characterized in that** the topical compositions are useful for fighting localized excess weight.

3. Use according to Claim 1, **characterized in that** the topical compositions are useful for refining the silhouette, accelerating the removal of excesses, better defining the contour of the body and/or resculpturing the silhouette.

4. Use according to any one of Claims 1 to 3,
**characterized in that** one or more isoflavones and also one or two slimming active agents of lipolytic type and/or one or two slimming active agents of liporeducing type are applied, simultaneously, separately or sequentially over time.

5. Use according to Claim 4, **characterized in that** the slimming active agent of lipolytic type is chosen from: caffeine, rhodysterol, palmitoylcarnitine, alpha and gamma bioactive agents, escin, ginkgo biloba and sphingosine.

6. Use according to Claim 4 or 5, **characterized in that** the slimming active agent of liporeducing type is chosen from: *andiroba, Garcinia cambogia* and rutin.

7. Use according to any one of Claims 1 to 6,
**characterized in that** one or two anti-infiltration or venotonic active agents chosen from: viburnum, ivy, arnica, mouseear hawkweed, wild pansy, *Fucus vesiculosus, Ruscus*, Ginkgo biloba and escin, are also applied simultaneously, separately or sequentially over time.

8. Use according to any one of Claims 1 to 7,
**characterized in that** an extract of *Centella asiatica* and/or soy proteins is also applied simultaneously, separately or sequentially over time.

9. Use according to any one of Claims 1 to 8,
**characterized in that** the isoflavones are chosen from: genistein, daidzein, glycitein or a mixture thereof; these isoflavones being in aglycone or glycosylated form.

10. Use according to Claim 8, **characterized in that** the genistein is of plant origin from soy.

11. Cosmetic treatment method for promoting slimming, **characterized in that** a cosmetic composition comprising one or more isoflavones in aglycone or glycosylated form and an extract of *Sophora j*aponica flowers is applied topically.

12. Cosmetic treatment method for fighting localized excess weight, **characterized in that** a composition comprising one or more isoflavones, in aglycone or glycosylated form, and an extract of *Sophora japonica* flowers is applied topically.

13. Cosmetic treatment method for refining the silhouette, accelerating the removal of excesses, better defining the contour of the body and/or resculpturing the silhouette, **characterized in that** a composition comprising one or more isoflavones, in aglycone or glycosylated form, and an extract of *Sophora japonica* flowers is applied topically.

14. Cosmetic treatment method according to any one of Claims 11 to 13, **characterized in that** one or more isoflavones and the active agent(s) as defined in any one of claims 4 to 8 are applied topically to the areas of skin liable to form localized excess weight, simultaneously, separately or sequentially over time.

15. Cosmetic treatment method according to any one of Claims 11 to 14, **characterized in that** the isoflavones are chosen from: genistein, daidzein, glycitein or a mixture thereof; these isoflavones being in aglycone or glycosylated form.

16. Cosmetic treatment method according to Claim 15, **characterized in that** the genistein is of plant origin from soy.

17. Cosmetic treatment method according to any one of Claims 11 to 16, **characterized in that** pregnant women or women who have given birth within 6 months are treated.

## Patentansprüche

1. Verwendung von Isoflavonen in aglyconer oder glycosylierter Form und eines Extrakts aus Blüten von Sophora Japonica für die Zubereitung topischer Zusammensetzungen, die zur Förderung des Schlankheitseffekts zweckdienlich sind.

2. Verwendung nach Anspruch 1, **dadurch gekennzeichnet, dass** die topischen Zusammensetzungen zur Bekämpfung örtlichen Übergewichts zweckdienlich sind.

3. Verwendung nach Anspruch 1, **dadurch gekennzeichnet, dass** die topischen Zusammensetzungen zur Verfeinerung der Silhouette, zur beschleunigten Beseitigung von Überschüssen, zur besseren Definition der Kontur des Körpers und/oder Neumodellierung der Silhouette zweckdienlich sind.

4. Verwendung nach einem der Ansprüche 1 bis 3, **dadurch gekennzeichnet, dass** ein oder mehrere Isoflavone sowie ein oder zwei schlankmachende Wirkstoffe vom lipolytischen Typ und/oder ein oder zwei schlankmachende Wirkstoffe vom fettreduzierenden Typ gleichzeitig, getrennt oder zeitversetzt angewendet werden.

5. Verwendung nach Anspruch 4, **dadurch gekennzeichnet, dass** der schlankmachende Wirkstoff vom lipolytischen Typ aus dem Koffein, dem Rhodysterol, dem Palmitoylcarnitin, den Bioaktiva Alpha und Gamma, dem Escin, Ginkgo biloba und dem Sphingosin ausgewählt ist.

6. Verwendung nach Anspruch 4 oder 5, **dadurch gekennzeichnet, dass** der schlankmachende Wirkstoff vom fettreduzierenden Typ aus *Andiroba, Garcinia Cambogia* und dem Rutin ausgewählt ist.

7. Verwendung nach einem der Ansprüche 1 bis 6,
**dadurch gekennzeichnet, dass** weiterhin ein oder zwei drainierende oder verienstärkende Wirkstoffe, die aus dem Viburnum, dem Efeu, der Arnika, dem Habichtskraut, dem Wilden Stiefmütterchen, *Fucus vesiculosus, Ruscus,* Ginkgo biloba und dem Escin ausgewählt sind, gleichzeitig, getrennt oder zeitversetzt angewendet werden.

8. Verwendung nach einem der Ansprüche 1 bis 7,
**dadurch gekennzeichnet, dass** weiterhin ein Extrakt von *Centella Asiatica* und/oder Sojaproteine gleichzeitig, getrennt oder zeitversetzt angewendet wird.

9. Verwendung nach einem der Ansprüche 1 bis 8,
**dadurch gekennzeichnet, dass** aus dem Genistein, dem Daidzein, dem Glycitein oder einem Gemisch dieser als Isoflavone ausgewählt wird, wobei diese in aglyconer oder glycosylierter Form sind.

10. Verwendung nach Anspruch 8, **dadurch gekennzeichnet, dass** das Genistein pflanzlicher Herkunft aus Soja ist.

11. Methode der kosmetischen Behandlung zur Förderung des Schlankheitseffekts, **dadurch gekennzeichnet, dass** eine kosmetische Zusammensetzung, die ein oder mehrere Isoflavone in aglyconer oder glycosylierter Form und einen Extrakt aus Blüten von Sophora Japonica umfasst, auf topischem Weg angewendet wird.

12. Methode der kosmetischen Behandlung zur Bekämpfung örtlichen Übergewichts, **dadurch gekennzeichnet, dass** eine Zusammensetzung, die ein oder mehrere Isoflavone in aglyconer oder glycosylierter Form und einen Extrakt aus Blüten von Sophora Japonica umfasst, auf topischem Weg angewendet wird.

13. Methode der kosmetischen Behandlung zur Verfeinerung der Silhouette, beschleunigten Beseitigung von Überschüssen, besseren Definition der Kontur des Körpers und/oder Neumodellierung der Silhouette,
**dadurch gekennzeichnet, dass** eine Zusammensetzung, die ein oder mehrere Isoflavone in aglyconer oder glycosylierter Form und einen Extrakt aus Blüten von Sophora Japonica umfasst, auf topischem Weg angewendet wird.

14. Methode der kosmetischen Behandlung nach einem der Ansprüche 11 bis 13, **dadurch gekennzeichnet, dass** auf die Bereiche der Haut, die imstande sind, örtlich Übergewicht auszubilden, ein oder mehrere Isoflavone sowie der oder die Wirkstoffe wie in einem der Ansprüche 4 bis 8 definiert gleichzeitig, getrennt oder zeitversetzt auf topischem Weg angewendet werden.

15. Methode der kosmetischen Behandlung nach einem der Ansprüche 11 bis 14, **dadurch gekennzeichnet, dass** aus dem Genistein, dem Daidzein, dem Glycitein oder einem Gemisch dieser als Isoflavone ausgewählt wird, wobei diese in aglyconer oder glycosylierter Form sind.

16. Methode der kosmetischen Behandlung nach Anspruch 15, **dadurch gekennzeichnet, dass** das Genistein pflanzlicher Herkunft aus Soja ist.

17. Methode der kosmetischen Behandlung nach einem der Ansprüche 11 bis 16, **dadurch gekennzeichnet, dass** Schwangere oder Frauen behandelt werden, die seit weniger als 6 Monaten entbunden haben.
